# FASCICULE DE BREVET EUROPEEN

(11) **EP 1 296 600 B1**
(45) Date de publication et mention de la délivrance du brevet: **02.11.2006**
(21) Numéro de dépôt: 01949603.3
(22) Date de dépôt: 02.07.2001
(51) Int. Cl.: A61B 17/28, A61B 10/00

(54) **PINCE MULTIFONCTIONS A USAGE MEDICAL COMPORTANT DEUX MACHOIRES ARTICULEES**
MULTIFUNKTIONELLE MEDIZINISCHE ZANGE MIT ZWEI SCHWENKBAREN BACKEN
MULTIPURPOSE CLAMP FOR MEDICAL USE COMPRISING TWO ARTICULATED JAWS

(30) Priorité: 05.07.2000 FR 0008858
(43) Date de publication de la demande: 02.04.2003
(73) Titulaire: Flipo, Bernard, 06100 Nice (FR)
(72) Inventeur: Flipo, Bernard, 06100 Nice (FR)
(74) Mandataire: Hautier, Jean-Louis
(86) Numéro de dépôt international: PCT/FR2001/002106
(87) Numéro de publication internationale: WO 2002/002019

(56) Documents cités:
- DE-A- 3 023 671
- DE-U- 9 005 519
- DE-U- 9 413 527
- DE-U- 29 810 758
- US-A- 1 462 202
- US-A- 2 566 626
- US-A- 3 404 677
- US-A- 4 597 385
- US-A- 5 052 402
- US-A- 5 667 526

## Description

La présente invention concerne une pince multifonctions à usage médical.

Elle trouvera son application dans le domaine de l'usage et de la fabrication de pinces à destination médicale particulièrement dans le domaine gynécologique.

Les pinces sont communément dotées de deux mâchoires articulées par pivot et commandées par le praticien depuis une zone de préhension.

Dans le domaine gynécologique, particulièrement, on a l'habitude d'utiliser différentes pinces remplissant chacune une fonction spécifique. Ainsi, sont connues des pinces porte-coton, dont les mâchoires présentent des faces internes sensiblement planes, et pourvues de stries parallèles transversales afin de permettre la préhension de certains accessoires, de cotons, ou encore des pansements.

On utilise aussi fréquemment des pinces dites de préhension de col.

D'aspect général identique aux pinces précédemment présentées, les pinces de préhension de col comportent des mâchoires dotées de plusieurs griffes à leur extrémité.

On peut, par le biais de cette griffe maintenir et/ou écarter des tissus tels que ceux du col de l'utérus.

Des pinces particulièrement connues à ce sujet portent le nom de pinces de MUSEUX, et pinces de POZZI.

On utilise encore de façon quotidienne dans le domaine gynécologique des pinces à polypes.

La face interne de leurs mâchoires est excavée formant un organe de réception tel une cuillère.

Au moyen de ces cavités, il est possible de prélever différents éléments et notamment des polypes.

Le document US-A-5.667.526 montre une pince, correspondant au préambule de la revendication 1, de forme très particulière et dotée de dents fortement agressives. Elle n'est utilisable que pour certaines fonctions limitées et son actionnement complexe rend impossible sa fabrication à faible coût.

On connaît aussi du document US-A-4.597.385 une pince à biopsie dotée d'un organe de coupe à l'avant de l'une des mâchoires et de deux dents de rétention des tissus. Cette pince est elle aussi, d'intérêt limité puisqu'elle ne s'applique qu'à la réalisation de biopsie. Sa conformation est aussi complexe de par la conception des mâchoires.

Ces différentes pinces donnent individuellement satisfaction d'une manière générale.

Cependant, on constate qu'il est nécessaire, pour chaque patiente, d'avoir recours à ces trois pinces de façon cumulative.

Le praticien est donc obligé de disposer de multiples jeux de ces différentes pinces pour diverses patientes successives.

Outre le coût d'achat, une telle organisation impose d'importants frais de stérilisation des pinces.

Une étape de stérilisation des pinces est systématique après leur usage.

Or, les installations de stérilisation sont généralement coûteuses, surtout lorsqu'elles sont ramenées à l'exploitation individuelle d'un praticien exerçant en profession libérale.

En outre, la stérilisation doit être réalisée par une personne ayant les compétences adéquates, ce qui engendre également un coût important en terme de main d'oeuvre.

Les stérilisations, bien que très performantes, ne suppriment jamais tout risque de défaut dans la stérilisation obtenue.

Il existe donc un besoin important de proposer des pinces permettant de limiter le jeu de pinces nécessaires aux gynécologues, et, de façon préférée, d'éviter, le recours à la stérilisation.

La présente invention s'inscrit dans ce cadre et permet de remédier aux inconvénients des pinces actuellement sur le marché.

L'un des premiers buts de l'invention est de réaliser une pince réalisant plusieurs fonctions parmi celles existant actuellement pour des pinces distinctes.

En ce sens, la pince ici présentée, a l'avantage de remplir simultanément les fonctions de pince porte-coton, de pince de préhension de col et de pince à polypes.

Par ailleurs, le fait de réduire le nombre de pinces permet d'envisager leur caractère jetable.

A ce sujet, la pince selon l'invention pourra être réalisée à moindre coût de par sa conception et sa configuration et, éventuellement, en matière plastique.

Par conséquent, l'invention associe le caractère pratique de l'emploi d'une seule pince, à l'aspect économique des instruments jetables ne nécessitant pas de stérilisations multiples.

Un autre avantage de l'invention est de réaliser une pince multifonctions tout en conservant son efficacité pleine et entière pour la réalisation de chacune de ces fonctions, sans l'usure du temps que l'on connaît pour des pinces classiques non jetables.

Selon une variante préférée, la pince comporte des branches incurvées de telle sorte à exercer un effort supplémentaire sur les mâchoires, après leurs mise en contact, par déformation élastique des branches. La forcipressure est ainsi grandement favorisée.

D'autres buts et avantages apparaîtront au cours de la description qui suit, qui n'est cependant pas limitative de l'invention.

La présente invention concerne une pince multifonctions à usage médical comportant deux mâchoires articulées par pivot et commandées par le praticien depuis une zone de préhension, comme décrit dans la première revendication.

Cette pince pourra être réalisée selon les diverses variantes introduites ci-après :
- les alvéoles sont configurées pour créer une réservation fermée en forcipressure.
- les alvéoles ont une section elliptique dont le grand axe est orienté suivant l'axe longitudinal de la mâchoire.
- les alvéoles s'étendent jusqu'à l'extrémité distale des mâchoires entre les deux griffes.
- la zone de préhension est formée par deux branches à anneaux reliées par une entablure réalisant le pivot des mâchoires, lesdites mâchoires étant chacune formée dans le prolongement d'une branche du côté de l'entablure opposée à la zone de préhension.
- la partie postérieure des branches, située du côté de l'entablure opposée à la zone de préhension, est incurvée de façon convexe et est en matériau déformable élastiquement lors de l'appui des mâchoires.
- la courbure des deux parties postérieures est symétrique.
- la partie antérieure des branches, située du même côté de l'entablure que la zone de préhension, est incurvée de façon concave et est en matériau déformable élastiquement lors de l'appui des mâchoires.
- la courbure des deux parties antérieures est symétrique.
- la distance entre l'extrémité distale des mâchoires et l'entablure est de 7 à 9 cm.
- elle est constituée en matière plastique.

Les dessins ci-joints sont donnés à titre d'exemples indicatifs et non limitatifs. Ils représentent un mode de réalisation. Ils permettront de comprendre aisément l'invention.

La figure 1 est une vue partielle de côté de l'extrémité d'une pince dans un mode particulier de l'invention.

La figure 2 est une vue générale d'un mode de réalisation de la pince multifonctions de la pince ici présentée.

La figure 3 est une vue de côté partielle des mâchoires d'une pince en position ouverte.

La figure 4 est une vue de dessous d'une des mâchoires de la pince selon l'invention.

La figure 5 est une vue de dessus de l'autre mâchoire selon une possibilité de réalisation.

La figure 6 illustre une conformation préférée des branches de la pince, les mâchoires étant ouvertes.

La figure 7 est une vue mâchoires fermées d'une autre variante de conformation de la pince.

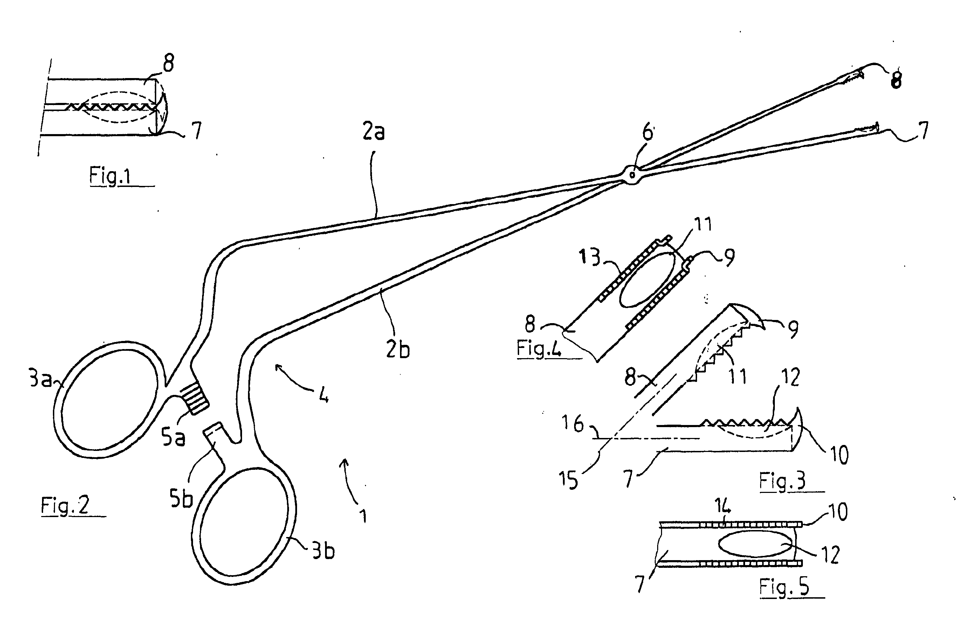

La figure 2 montre une vue générale d'un exemple de réalisation de la pince ici présentée.

Suivant cet exemple préféré, la pince comporte une zone de préhension 1 sensiblement à l'une de ses extrémités formée par deux branches allongées repérées 2a, 2b, et dotées d'anneaux à leurs extrémités, repérés 3a, 3b.

Les anneaux permettent la préhension par le praticien afin de commander la fermeture ou l'ouverture de la pince.

Selon l'exemple illustré, les branches 2a, 2b sont reliées par une entablure 6 de façon courante dans le domaine technique considéré actuellement.

Par ailleurs, telles que représentées en figure 2, les mâchoires 7, 8 que comportent la pince sont formées chacune dans le prolongement de l'une des branches 2a, 2b de l'autre côté de l'entablure 6 par rapport à la zone de préhension 1.

En se référant à la disposition relative des mâchoires en figure 2, on appellera mâchoire inférieure, la mâchoire repérée 7, et la mâchoire supérieure, la mâchoire repérée 8.

Toute autre forme de pince est bien entendu envisageable selon l'invention, et la disposition particulière de la figure 2 n'est donnée qu'à titre indicatif.

De même, la partie coudée 4 que présentent les branches 2a, 2b n'est pas systématique, et on aurait pu envisager une pince dont les deux branches sont sensiblement rectilignes.

Pour permettre la réalisation d'une pince multifonctions, les mâchoires 7, 8 présentent des moyens spécifiques.

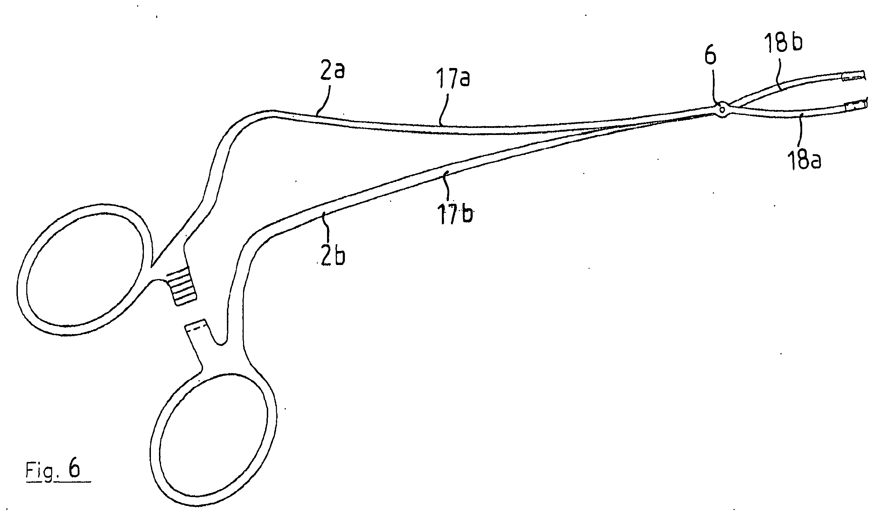

Ainsi, chaque mâchoire 7, 8 comprend sur sa face interne une alvéole 11, 12 comme présentées respectivement aux figures 4 et 5.

La formation de telles alvéoles 11, 12 permet de créer une réservation à l'intérieur des mâchoires 7, 8 lorsque celles-ci sont en position jointive, éventuellement en forcipressure.

A titre indicatif, mais préféré, les alvéoles 11, 12 ont une section elliptique dont le grand axe est orienté suivant l'axe longitudinal de la mâchoire 7, 8.

Un faible écart entre le bord de l'alvéole 11, 12 et l'extrémité de la mâchoire 7, 8 est souhaitable, par exemple 1 mm, pour faciliter l'utilisation.

La longueur du grand axe des alvéoles 11, 12 pourra être par exemple de 15 mm.

De façon préférée les alvéoles 11, 12 ont une forme et des dimensions semblables et sont disposées de façon symétrique sur les mâchoires 7 et 8 afin de se faire face.

Ainsi, leurs ouvertures symétriques permettent de créer une réservation fermée lorsque les mâchoires sont jointives, les bords des ouvertures des alvéoles étant en contact dans cette position.

Le bord des ouvertures pourra dans ce cadre être au niveau de la mi-hauteur des stries 13, 14 présentées ci-après.

Les alvéoles 11, 12 ainsi formées permettront le prélèvement de tissus ou encore de polypes.

La pince ici présentée permet également la préhension et la manipulation de divers accessoires tels des cotons, fils de stérilet, .....

Elle réalise aussi une pince que l'on appelle communément pince porte-coton.

Pour ce faire, chaque mâchoire 7, 8 comprend sur sa face interne au moins une série de stries repérées 13 et 14 aux figures 4 et 5.

Telles que représentées, ces stries sont transversales par rapport à l'axe longitudinal 15, 16 des mâchoires 7, 8.

Par ailleurs, comme il ressort des figures 2 et 3, les stries 13, 14 disposées sur chacune des mâchoires 7, 8 sont complémentaires afin de s'imbriquer lorsque les mâchoires 7, 8 sont jointives comme en figure 1.

De façon préférée, on formera deux séries de stries 13, 14 sur chaque mâchoire 7, 8.

Plus précisément, ces deux séries de stries peuvent être disposées de part et d'autre de l'alvéole 11, 12.

Cette configuration ressort clairement des figures 4 et 5.

On notera qu'une telle disposition est particulièrement avantageuse en terme d'encombrement.

En effet, les stries 13, 14 ainsi réalisées remplissent parfaitement leurs fonctions d'accroche de différents accessoires sans pour autant occuper toute la largeur de la face interne des mâchoires 7, 8.

Par ailleurs, une disposition des séries de stries sensiblement à proximité des bords latéraux des mâchoires 7, 8 assure le bon maintien en position des mâchoires 7, 8 même si elles sont sollicitées en torsion lorsqu'elles sont jointives.

Par ailleurs, on constate que la forme elliptique préférée pour les alvéoles 11, 12 permet la formation d'une réservation d'un volume suffisant tout en préservant un espace latéral pour la formation des séries de stries 13, 14.

La pince selon l'invention remplit aussi la fonction de pince de préhension de col.

A cet effet, au moins une griffe 9, 10 est formée à l'extrémité distale de chaque mâchoire 7, 8.

Dans l'exemple représenté aux figures, on a formé deux griffes 9, 10 sur chaque mâchoire 7, 8.

Telles qu'apparaissant en figure 3, les griffes de chaque mâchoire 7, 8 sont orientées vers l'autre mâchoire.

Par ailleurs, lorsque les mâchoires 7, 8 sont en position jointives, les griffes 9, 10 des mâchoires 7, 8 sont telles qu'elles s'engrènent. De cette façon, elles ne gênent pas la position fermée des mâchoires 7, 8.

Un exemple de réalisation des griffes 9, 10 s'engrénant est présenté en figure 1.

Par mesure de commodité pour le praticien, les alvéoles 11, 12 s'étendent préférentiellement jusqu'à l'extrémité distale des mâchoires 7, 8 entre les griffes 9, 10. De cette façon, la réservation pour la réception de polype est située à proximité de l'extrémité de la pince.

Toujours par commodité d'usage et à titre d'exemple, la distance entre l'extrémité distale des mâchoires 7, 8 et l'entablure 6 pourra être de 7 à 9 centimètres.

Par ailleurs, on pourra former des pattes de fermeture 5a, 5b au niveau de la zone de préhension 1.

Par ces moyens, il sera possible de maintenir la pince en position fermée avec, éventuellement une légère contrainte de fermeture permanente.

Des pattes de fermeture 5a et 5b telles que représentées en figure 2 et de conception courante pourront être mises en oeuvre.

On constate par la présente description et l'illustration des dessins que la pince selon l'invention remplit plusieurs fonctions de façon tout aussi efficace que des pinces individuellement prévues pour remplir chacune de ces fonctions.

Par ailleurs, cette réalisation est effectuée sans pour autant avoir recours à une conception complexe qui grèverait le coût de réalisation des pinces.

En outre, la configuration de ces pinces est telle qu'elle peut être réalisée en matière plastique et, par exemple, par des étapes de moulage.

De cette façon, on peut réaliser des pinces de faible coût qui pourront être destinées à un usage unique.

Particulièrement, pour une réalisation en matière plastique, il est important de préserver des caractéristiques optimales en forcipressure, lorsque les mâchoires 7, 8 sont fermées.

Pour ce faire, on propose préférentiellement de donner une forme incurvée aux branches 2a, 2b, permettant une déformation élastique des branches lorsque les mâchoires sont en contact, pour augmenter l'effort appliqué.

On utilisera un matériau dont les propriétés de déformation élastique sont en rapport avec le niveau de déformation souhaité pour les branches 2a, 2b.

La courbure donnée aux branches 2a, 2b est inverse à celle qu'elle a tendance à adopter lorsque la force l'appui des mâchoires 7, 8.

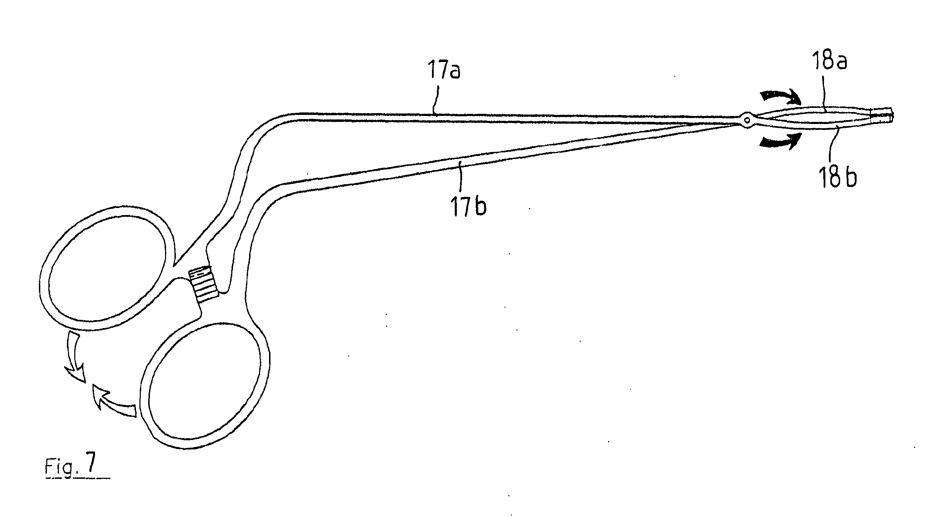

En se référant à la figure 7, la forme des parties postérieures 18a, 18b des branches 2a, 2b (ces parties sont celles situées du côté opposé à la zone de préhension 1) ayant une courbure convexe, préférentiellement de façon symétrique.

Les flèches pleines en figure 7 illustre le sens de la déformation élastique qui a tendance à annuler la courbure convexe.

De façon supplémentaire ou alternative, les parties antérieures 17a, 17b des branches 2a, 2b (ces parties sont du même côté que la zone de préhension 1) ont une courbure concave, comme le montre la figure 6.

La réalisation d'une telle pince à branches courbées telles qu'en figure 6 et 7 est possible par ailleurs avec tout type et conception de mâchoires.

### REFERENCES

1. Zone de préhension
2a, 2b - Branches
3a, 3b - Anneaux
4. Coude
5a, 5b - Pattes de fermetures
6. Entablure
7. Mâchoire inférieure
8. Mâchoire supérieure
9. Griffes supérieures
10. Griffes inférieures
11. Alvéole supérieure
12. Alvéole inférieure
13. Stries supérieures
14. Stries inférieures
15. Axe longitudinal de la mâchoire supérieure
16. Axe longitudinal de la mâchoire inférieure
17a, 17b. Partie antérieure
18a, 18b. Partie postérieure

## Revendications

1. Pince à usage médical comportant deux mâchoires (7,8) articulées par pivot et commandées par le praticien depuis une zone de préhension (1), dont chaque mâchoire (7, 8) comprend, sur sa face interne, une alvéole (11, 12) telle que les deux alvéoles (11, 12) forment une réservation lorsque les mâchoires (7, 8) sont jointives, **caractérisée par le fait**
**que**
- chaque mâchoire (7, 8) comprend deux séries de stries (13, 14) transversales formées de part et d'autre de l'alvéole (11, 12) suivant la direction de l'axe longitudinal (15, 16) de la mâchoire (7, 8), lesdites séries de stries (13, 14) étant complémentaires pour s'imbriquer lorsque les mâchoires (7, 8) sont jointives,
- chaque mâchoire (7, 8) comprend deux griffes (9, 10) à son extrémité distale, orientée vers l'autre mâchoire (7, 8), les griffes (9, 10) des mâchoires (7, 8) engrénant en position jointive,
- les alvéoles (11, 12) ont des ouvertures symétriques dont les bords sont en contact lorsque les mâchoires (7, 8) sont jointives, pour créer une réservation fermée,
de sorte à former une pince multifonctions.

2. Pince multifonctions, selon la revendication 1, dans laquelle les alvéoles (11, 12) sont configurées pour créer une réservation fermée en forcipressure.

3. Pince multifonctions, selon l'une quelconque des revendications 1 ou 2, **caractérisée par le fait**
**que** les alvéoles (11, 12) ont une section elliptique dont le grand axe est orienté suivant l'axe longitudinal (15, 16) de la mâchoire (7, 8).

4. Pince multifonctions, selon la revendication 3, **caractérisée par le fait**
**que** les alvéoles (11, 12) s'étendent jusqu'à l'extrémité distale des mâchoires (7, 8) entre les deux griffes (9, 10).

5. Pince multifonctions, selon l'une quelconque des revendications 1 à 4, **caractérisée par le fait**
**que** la zone de préhension (1) est formée par deux branches (2a, 2b) à anneaux (3a, 3b) reliées par une entablure (6) réalisant le pivot des mâchoires (7, 8), lesdites mâchoires (7, 8) étant chacune formée dans le prolongement d'une branche (2a, 2b) du côté de l'entablure (6) opposée à la zone de préhension (1).

6. Pince multifonctions, selon la revendication 5, **caractérisée par le fait**
**que** la partie postérieure (18a, 18b) des branches (2a, 2b), située du côté de l'entablure (6) opposée à la zone de préhension (1), est incurvée de façon convexe et est en matériau déformable élastiquement lors de l'appui des mâchoires (7, 8).

7. Pince multifonctions, selon la revendication 6, **caractérisée par le fait**
**que** la courbure des deux parties postérieures (18a, 18b) est symétrique.

8. Pince multifonctions, selon l'une quelconque des revendications 5 à 7, **caractérisée par le fait**
**que** la partie antérieure (17a, 17b) des branches (2a, 2b), située du même côté de l'entablure (6) que la zone de préhension (1), est incurvée de façon concave et est en matériau déformable élastiquement lors de l'appui des mâchoires (7, 8).

9. Pince multifonctions, selon la revendication 8, **caractérisée par le fait**
**que** la courbure des deux parties antérieures (17a, 17b) est symétrique.

10. Pince multifonctions, selon l'une quelconque des revendications 5 à 10, **caractérisée par le fait**
**que** la distance entre l'extrémité distale des mâchoires (7, 8) et l'entablure (6) est de 7 à 9 cm.

11. Pince multifonctions, selon l'une quelconque des revendications 1 à 10, **caractérisée par le fait**
**qu'**elle est constituée en matière plastique.

## Claims

1. Clamp for medical use comprising two jaws (7, 8) articulated on a pivot and operated by the user from a gripping part (1), each jaw (7, 8) of which has a cavity (11, 12) on its inner face, arranged so that the two cavities (11, 12) form a hollow when jaws (7, 8) are brought together, **characterised in that**
- each jaw (7, 8) includes two series of transversal striations (13, 14) formed on both sides of cavity (11, 12) on longitudinal axis (15, 16) of jaw (7, 8), the aforementioned striations (13, 14) being complementary so that they fit ones into the others when jaws (7, 8) are brought together,
- each jaw (7, 8) has two claws (9, 10) at its distal end, each jaw being oriented towards the other jaw (7, 8), the claws (9, 10) of jaws (7, 8) meshing when in the closed position,
- cavities (11, 12) have symmetrical openings, the edges of which are in contact when jaws (7, 8) are closed in order to create a closed hollow, so as to form a multipurpose clamp.

2. Multipurpose clamp according to claim 1, in which cavities (11, 12) are arranged so as to create a hollow closed by forcipressure.

3. Multipurpose clamp according to any of claims 1 or 2, **characterised in that** cavities (11, 12) have an elliptical section the long axis of which is oriented along longitudinal axis (15, 16) of jaws (7, 8).

4. Multipurpose clamp according to claim 3, **characterised in that**
cavities (11, 12) extend up to the distal end of jaws (7, 8) between two claws (9,10).

5. Multipurpose clamp according to any of claims 1 to 4, **characterised in that**
gripping part (1) is formed by two arms (2a, 2b) with rings (3a, 3b) connected by a pivot (6) on which jaws (7, 8) articulate, each of aforementioned jaws (7, 8) being formed in the extension of an arm (2a, 2b) on the side of pivot (6) opposite gripping part (1).

6. Multipurpose clamp according to claim 5, **characterised in that**
rear part (18a, 18b) of arms (2a, 2b) positioned on the side of pivot (6) opposite to gripping part (1) is curved convexly and is made from a material which is elastically deformable when pressure is applied on jaws (7, 8).

7. Multipurpose clamp according to claim 6, **characterised in that**
the curvature of the two rear parts (18a, 18b) is symmetrical.

8. Multipurpose clamp according to any of claims 5 to 7, **characterised in that**
forward part (17a, 17b) of arms (2a, 2b), located on the same side of pivot (6) as gripping part (1), is curved inwards in a concave manner and is made of a material which is elastically deformable when pressure is applied on jaws (7, 8).

9. Multipurpose clamp according to claim 8, **characterised in that**
the curvature of the two forward parts (17a, 17b) is symmetrical.

10. Multipurpose clamp according to any of claims 5 to 10, **characterised in that**
the distance between the distal end of jaws (7, 8) and pivot (6) is 7 to 9 cm.

11. Multipurpose clamp according to any of claims 1 to 10, **characterised in that** it is made of plastic.

## Patentansprüche

1. Zange für den medizinischen Gebrauch mit zwei Backen (7,8), die gelenkig verbunden sind und vom Arzt mittels einer Greifzone (1) betätigt werden, bei der jede Backe (7, 8) auf ihrer Innenseite eine Zelle (11, 12) besitzt, derart, dass die beiden Zellen (11, 12), wenn sie zusammengelegt werden, eine Aufnahme bilden, **gekennzeichnet dadurch, dass**
- in jeder Backe (7, 8) beidseitig jeder Zelle (11, 12) zwei zur Längsachse (15, 16) der Backe (7, 8) querliegende Zahnreihen (13, 14) ausgebildet sind, und diese Zahnreihen komplementär sind und ineinander eingreifen, wenn die Backen (7, 8) aufeinander liegen,
- jede Backe (7, 8) am distalen Ende zwei Greifer (9, 10) besitzt, die in Richtung der anderen Backe (7, 8) zeigen und die Greifer (9, 10) der Backen (7, 8) in geschlossener Stellung ineinander eingreifen,
- die Zellen (11, 12) symmetrische Öffnungen besitzen, deren Ränder miteinander in Kontakt sind, wenn die Backen (7, 8) aufeinander liegen und eine geschlossene Aufnahme bilden,
so dass eine Mehrzweckzange entsteht.

2. Mehrzweckzange gemäss Anspruch 1, in welcher die Zellen (11, 12) so ausgebildet sind, dass beim Abklemmen von Arterien eine geschlossene Aufnahme gebildet wird.

3. Mehrzweckzange gemäss einem der Ansprüche 1 oder 2, **gekennzeichnet dadurch,**
**dass** die Zellen (11, 12) einen elliptischen Querschnitt besitzen, dessen große Achse in Längsrichtung (15, 16) der Backe (7, 8) zeigt.

4. Mehrzweckzange gemäss Anspruch 3, **gekennzeichnet dadurch,**
**dass** die Zellen (11, 12) zwischen den beiden Greifern (9, 10) bis zum distalen Ende der Backen (7, 8) reichen.

5. Mehrzweckzange gemäss einem der Ansprüche 1 bis 4, **gekennzeichnet dadurch,**
**dass** die Greifzone (1) aus zwei mit Ringen (3a, 3b) versehenen Griffen (2a, 2b) besteht, die an einer Abflachung (6), welche das Gelenk der Backen (7, 8) darstellt, miteinander verbunden sind, und bei welcher jede der Backen (7, 8) in der Verlängerung eines Griffes (2a, 2b) auf der der Greifzone (1) in bezug auf die Abflachung (6) gegenüberliegenden Seite ausgebildet sind.

6. Mehrzweckzange gemäss Anspruch 5, **gekennzeichnet dadurch,**
**dass** der hintere Teil (18a, 18b) der Griffe (2a, 2b), der auf der der Abflachung (6) gegenüberliegenden Seite der Greifzone (1) liegt, konvex gebogen und aus einem Material ist, das beim Aufeinanderlegen der Backen (7,8) elastisch verformbar ist.

7. Mehrzweckzange gemäß Anspruch 6, **gekennzeichnet dadurch,**
**dass** die Krümmung der beiden hinteren Teile (18a, 18b) symmetrisch ist.

8. Mehrzweckzange gemäß einem der Ansprüche 5 bis 7, **gekennzeichnet dadurch,**
**dass** der hintere Teil (17a, 17b) der Griffe (2a, 2b), der in bezug auf die Abflachung (6) auf der gleichen Seite wie die Greifzone (1) liegt, konkav gebogen und aus einem Material ist, dass beim Aufeinanderlegen der Backen (7, 8) elastisch verformbar ist.

9. Mehrzweckzange gemäss Anspruch 8, **gekennzeichnet dadurch,**
**dass** die Krümmung der beiden hinteren Teile (17a, 17b) symmetrisch ist.

10. Mehrzweckzange gemäss einem der Ansprüche 5 bis 10, **gekennzeichnet dadurch,**
**dass** der Abstand zwischen dem distalen Ende der Backen (7, 8) und der Abflachung (6) 7 bis 9 cm beträgt.

11. Mehrzweckzange gemäß einem der Ansprüche 1 bis 10, **gekennzeichnet dadurch,**
**dass** sie aus Kunststoff ist.
